# EUROPEAN PATENT SPECIFICATION

(11) **EP 2 114 326 B1**
(45) Date of publication and mention of the grant of the patent: **19.03.2014**
(21) Application number: 07732080.2
(22) Date of filing: 21.03.2007
(51) Int. Cl.: A61F 5/56, A61F 5/08

(54) **APPARATUS FOR ASSISTING BREATHING THROUGH A PERSON'S NOSE**
GERÄT ZUR UNTERSTÜTZUNG DER ATMUNG DURCH DIE NASE EINER PERSON
APPAREIL POUR AIDER UNE PERSONNE À RESPIRER PAR LE NEZ

(30) Priority: 13.04.2006 GB 0607533
(43) Date of publication of application: 11.11.2009
(73) Proprietor: James, Phillipa, Whetstone London N20 0DZ (GB)
(72) Inventor: James, Lawrence, Christopher, William, Paphos 8042 (CY)
(74) Representative: Jones, Graham Henry
(86) International application number: PCT/GB2007/000988
(87) International publication number: WO 2007/119041

(56) References cited:
- EP-A- 0 958 798
- EP-A2- 0 824 015
- US-A- 4 778 466
- US-A- 5 775 335
- US-B1- 6 978 781

## Description

This invention relates to apparatus for assisting breathing through a person's nose. The apparatus may be used for assisting breathing generally, for example during the day or when a person is suffering from hay fever or a cold. The apparatus may also be used for reducing or eliminating snoring.

Many persons suffer from the problem of having difficulty in adequately breathing through their nose. A blocked nose during the day can cause much discomfort. A blocked nose during the night can lead to snoring when a person is asleep. Devices are known for inserting into a person's nose with a view to keeping nasal passages open as much as possible. These known devices are often uncomfortable to wear for lengthy periods of time and they may also look unattractive. EP-A-O 958 798 discloses a device which is for inserting into a person's nose and which comprises a first member for fitting in a first nostril of the nose, a second member for fitting in a second nostril of the nose, and a connecting member which connects the first and second members together. The first and second members are open loops. US-A 5 775 335 discloses a device which is for inserting into a person's nose and which is such that the first and second members are cylinders with longitudinally disposed ridges arranged around the outside of the cylinders. Devices are also known like sticking plaster which stick over the outside of a person's nose. Such devices tend to leave a white mark if they are worn for too long, especially if they are worn in the sun. They can also look unattractive.

It is an aim of the present invention to obviate or reduce the above mentioned problems.

Accordingly, the present invention provides apparatus for assisting breathing through a person's nose, which apparatus in use comprises a first member for fitting in a first nostril of the nose, a second member for fitting in a second nostril of the nose, and a connecting member which connects the first and second members together and which prevents the first and second members being pushed too far into the nose, characterised in that the first member comprises a strip which defines a closed loop, the second member comprises a strip which defines a closed loop, the connecting member comprises a strip which defines a concave shape, and the strip for the connecting member is larger in size in cross section than the size in cross section of the strips for the first and second members.

The apparatus of the invention may be one in which the first and second members extend upwardly from the connecting member and thereby further into the nostrils of the nose than ends of the connecting member from which the first and second members extend.

The apparatus may be one in which the closed loop is not a circular closed loop. The apparatus may alternatively be one in which the closed loop is a circular closed loop.

The apparatus may be such that the first and second members are first and second ring members.

Preferably, the apparatus is made from a plastics material or a synthetic rubber material. The material chosen for producing the apparatus should preferably be such that the apparatus is soft and flexible and thus comfortable to wear. A presently preferred plastics material is a silastic plastics material. Such a silastic plastics material is very soft and enables the apparatus to be a very comfortable fit in a person's nose.

Embodiments of the invention will now be described solely by way of example and with reference to the accompanying drawings in which:
Figure 1 is a perspective view of first apparatus which is not of the invention and which is for assisting breathing through a person's nose and ready for use;
Figure 2 shows the apparatus partially bent to the shape shown in Figure 1;
Figure 3 shows the apparatus in the form in which it is made and prior to the apparatus being bent to the shape shown in Figure 1;
Figure 4 is a section on the line A-A shown in Figure 2;
Figure 5 is a section on the line B-B shown in Figure 2;
Figure 6 is a section on the line C-C shown in Figure 1;
Figure 7 is a section on the line D-D shown in Figure 3;
Figure 8 shows the apparatus of Figures 1-7 fitted in a person's nose;
Figure 9 is a perspective view of second apparatus which is not of the invention and which is for assisting breathing through a person's nose;
Figure 10 is a top plan view of a first ring member forming part of the apparatus shown in Figure 9;
Figure 11 shows the apparatus of Figure 9 in the form in which it is made;
Figure 12 is a perspective view of third apparatus which is not of the invention and which is for assisting breathing through a person's nose;
Figure 13 is a top plan view of the apparatus shown in Figure 12;
Figure 14 is a perspective view of fourth apparatus which is not of the invention and which is for assisting breathing through a person's nose;
Figure 15 is a top plan view of the apparatus as shown in Figure 14;
Figure 16 is a front view of the apparatus as shown in Figure 14;
Figure 17 is a top view like Figure 15 but shows the apparatus in its use condition;
Figure 18 is a front view like Figure 16 but shows the apparatus in its use condition;
Figure 19 is a front perspective view of fifth apparatus which is not of the invention and which is for assisting breathing for a person's nose;
Figure 20 is a rear perspective view of the fifth apparatus as shown in Figure 19;
Figure 21 is a front view of the fifth apparatus as shown in Figure 19;
Figure 22 is a top view of the apparatus as shown in Figure 20 and in a large size;
Figure 23 is a top view of the apparatus as shown in Figure 19 and shown in a small size;
Figure 24 is an end view of the apparatus as shown in Figure 22;
Figure 25 is an end view of the apparatus as shown in Figure 23;
Figure 26 is a perspective view of sixth apparatus which is not of the invention and which is for assisting breathing through a person's nose;
Figure 27 is an underneath view of the apparatus shown in Figure 26;
Figure 28 is a view on arrow A shown in Figure 26;
Figure 29 is a view on arrow B shown in Figure 26; and
Figure 30 is a perspective view of seventh apparatus which is of the invention and which is for assisting breathing through a person's nose.

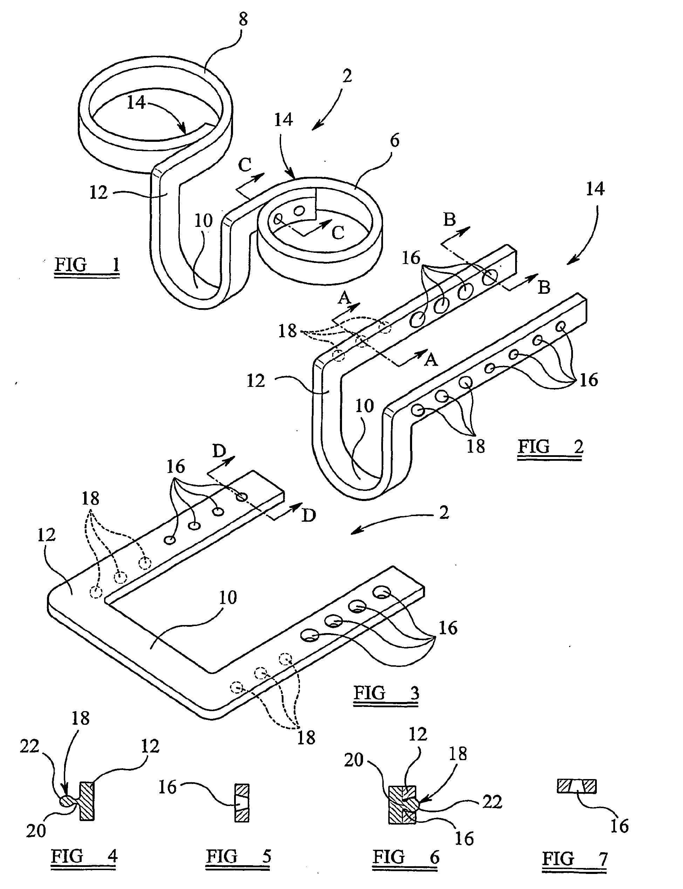

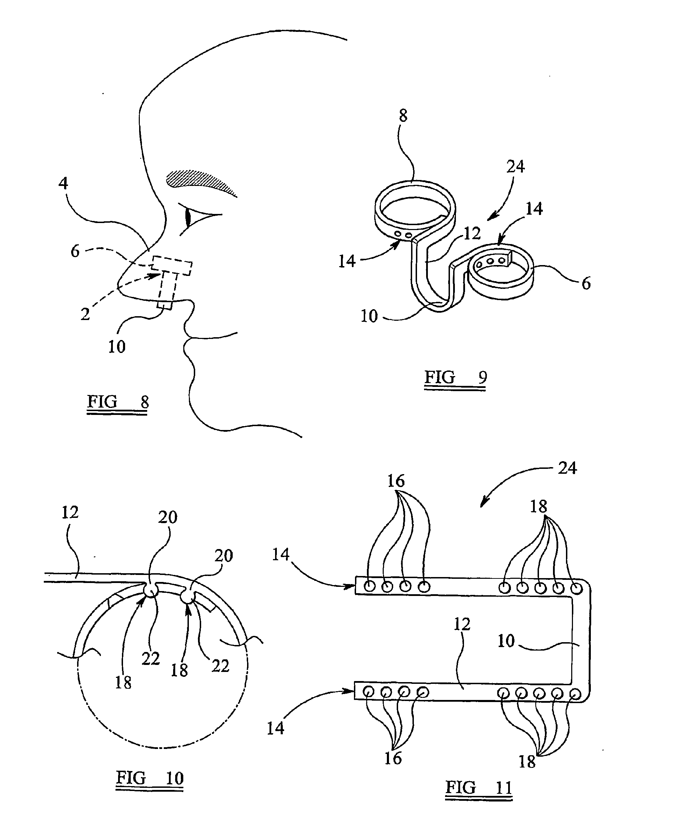

Referring to Figures 1 - 8 there is shown first apparatus 2 for assisting breathing through a person's nose 4. Figure 1 shows the shape of the apparatus 2 in use. As can be seen from Figure 1, the apparatus 2 in use comprises a first ring member 6 for fitting in a first nostril of the nose 4, a second ring member 8 for fitting in a second nostril of the nose 4, and a connecting member 10 which connects the first and the second ring members 6, 8 together. As can best be appreciated from Figure 8, the connecting member 10 also prevents the first and second ring members 6, 8 being pushed too far into the nose 4 because the connecting member 10 engages the septum of the nose 4.

The first and second ring members 6, 8 are adjustable in size for ensuring a comfortable fit in the nose 4. The apparatus 2 is such that it is made in flat form as shown in Figure 3 and it is then bent into the shape shown in Figure 1 for use. Figure 2 shows the apparatus 2 having been partially bent from the flat form shown in Figures 3 to the use position shown in Figure 1.

As can be seen from Figure 3, the apparatus 2 is made in flat form as a flat strip 12 of material. The flat strip 12 of material is U-shaped in plan. The apparatus 2 is such that the first and second ring members 6, 8 are adjustable by each having a stud and hole arrangement 14. Each stud and hole arrangement comprises a plurality of holes 16, and a plurality of studs 18. As best shown in Figures 2 and 3, there are four of the hole 16 and three of the studs 18. As shown in Figures 5 and 7, the holes 16 are frusto-conical holes. The shape of the studs 18 is shown in Figure 4. It will be seen that each stud 18 has a neck portion 20 which extends outwardly from the strip 12, and a head 22 which is located on the end of the neck portion 20 remote from the strip 12. The shape of the head 22 is designed to be an appropriate fit in the holes 16. Figure 6 shows one of the studs 18 located in one of the holes 16.

Referring now to Figures 9-11, there is shown second apparatus 24 for assisting breathing through a person's nose 4. Similar parts as in the apparatus 2 have been given the same reference numerals for ease of comparison and understanding. It will be seen from Figure 11 that the apparatus 24 has five of the studs 18 and four of the holes 14. It will also be seen from a comparison of Figures 1 and 9 that the strip 12 of material for producing the apparatus 24 is thinner than the strip 12 used for producing the apparatus 2.

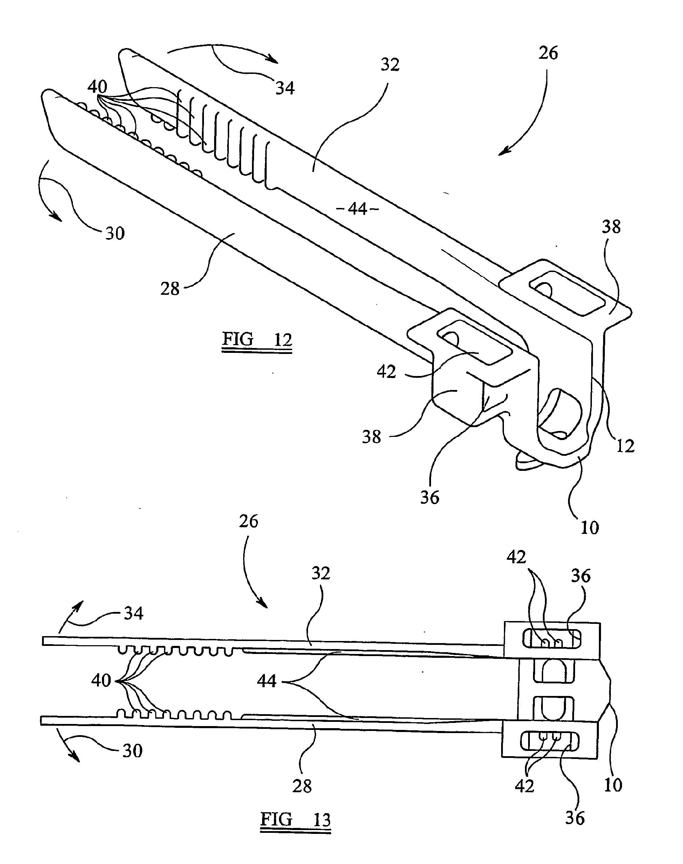

Referring now to Figures 12 and 13, there is shown third apparatus 26 for assisting breathing through a person's nose 4. Similar parts as in the apparatus 2 have again been given the same reference numerals for ease of comparison and understanding. The apparatus 26 is such that it is moulded to be more three dimensional than the apparatus 2. Also, the apparatus 26 is such that each ring member (not shown) is formed by bending an arm 28 in a direction shown by an arrow 30, and an arm 32 in a direction shown by an arrow 34. The free end of the arm 28 passes through an opening 36 in a housing 38. Teeth 40 on an inner face of the arm 28 are then able to engage teeth 42 on an inside face of the housing 38. The ring member formed by bending the arm 28 in the direction of the arrow 30 is adjustable in size by moving the teeth 40 relative to the teeth 42. The arm 32 is similarly bent into a ring member and adjusted in shape.

The arms 28, 32 have curved inner faces 44 as can best be appreciated from Figure 13. The formed first and second ring members are then very comfortable during use of the apparatus 26.

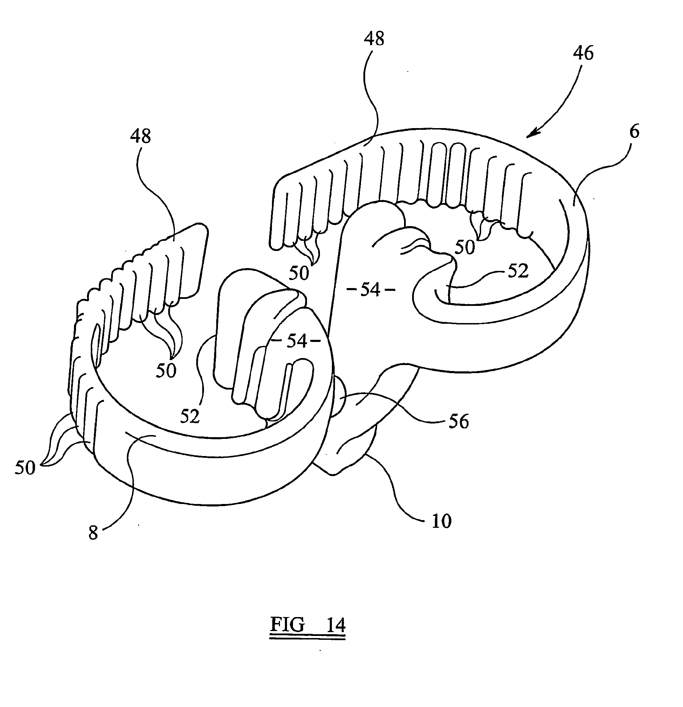

Referring to Figures 14-18, there is shown fourth apparatus 46 for assisting breathing through a person's nose. Similar parts as in previous Figures have been given the same reference numerals for ease of comparison and understanding.

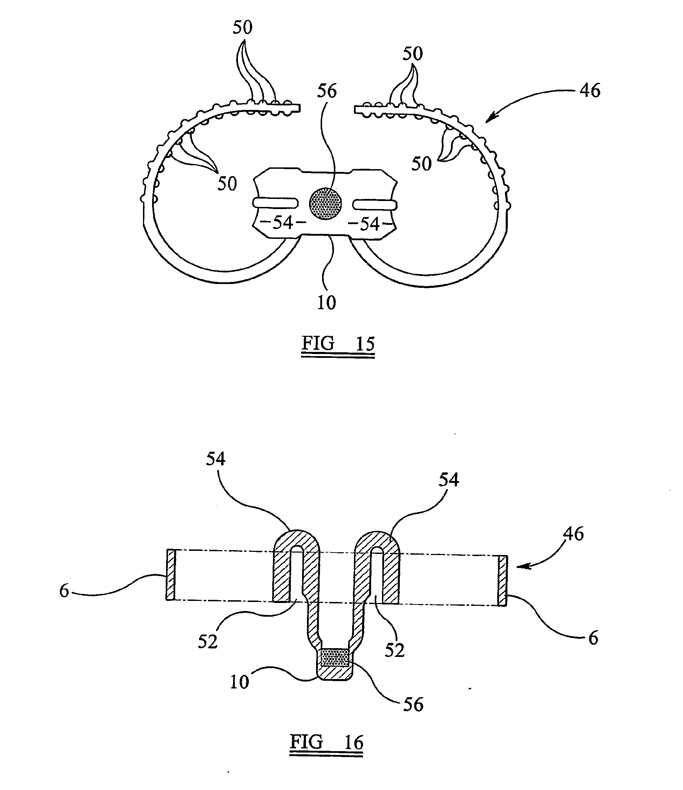

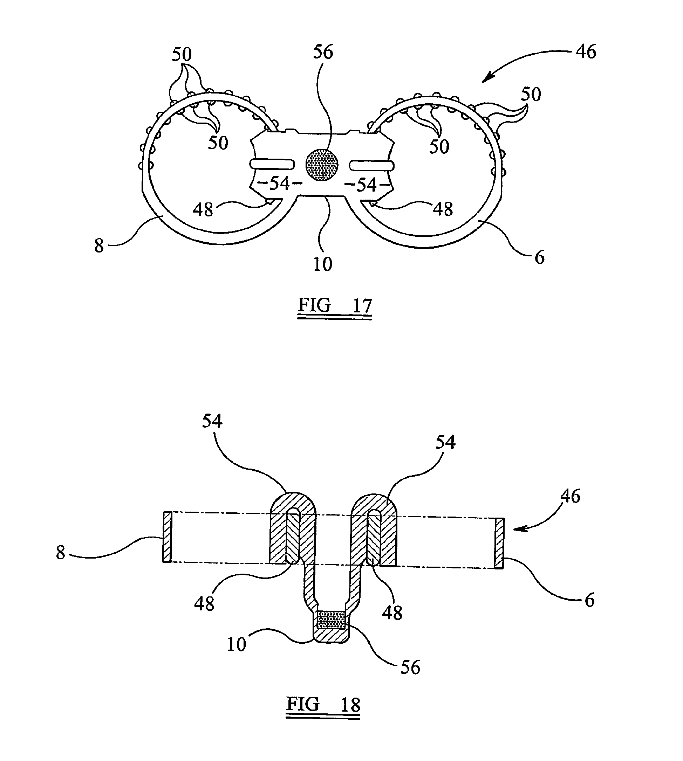

As can best be seen from Figures 14, 15 and 17, the first ring member 6 is provided with a toothed portion 48 having teeth 50. The toothed portion 48 pushes into a toothed complementarily-shaped aperture 52 in a side part 54 of the connecting member 10. Teeth defining sides of the aperture 52 engage on the teeth 50 so that the first ring member 6 can be made into a ring of any diameter as can be appreciated from a comparison of Figures 15 and 17. The second ring member 8 also has a toothed portion 48 with the teeth 50. Toothed portion 48 fits into an aperture 52 on a side part 54 of the second ring member 8. The second ring member 8 of appropriate diameter is formed in the same manner as with the first ring member 6.

It will be noted from Figures 14, 15 and 17 that the teeth 50 are on both sides of the toothed portion 48. The aperture 52 has teeth (not shown) on opposing sides. Thus the toothed portion 48 is easily and securely retained in its aperture 52.

The connecting member 10 is provided with a magnet 56. In an alternative embodiment of the invention (not shown) the magnet 56 may be omitted.

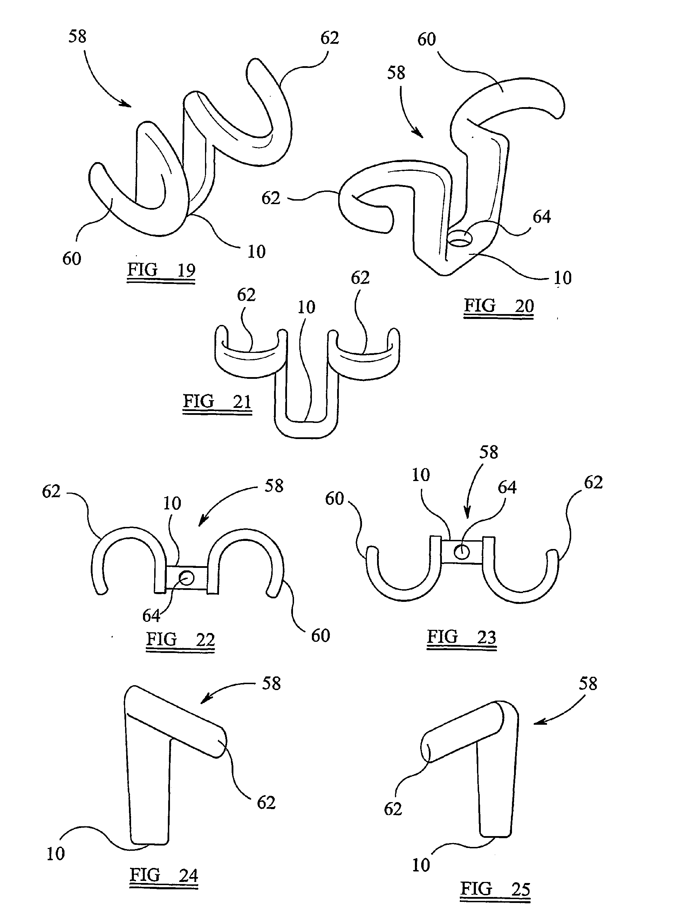

Referring now to Figures 19-23 there is shown fifth apparatus 58 for assisting breathing through a person's nose. Similar parts as in previous Figures have been given the same reference numerals for ease of comparison and understanding. The apparatus 58 is such that the connecting member 10 connects a first curved member 60 for fitting in a first nostril of the person's nose, and a second curved member 62 for fitting in a second nostril of the person's nose. The first and second curved members 60, 62 are like the first and second ring members except that they do not form complete rings. The connecting portion 10 is provided with an aperture 64. If desired, the aperture 64 may be omitted, or it may be provided with a magnet.

Figures 22 and 24 show what the apparatus 58 looks like in a large form. Figures 23 and 25 show what the apparatus 58 looks like in a small form. It will be noted that in both the large and the small forms, the curved members 60, 62 slope towards the connecting member 10. The curved members 60, 62 may be part circular, or part elliptical or curved to any appropriate shape for fitting in the nostrils of the person.

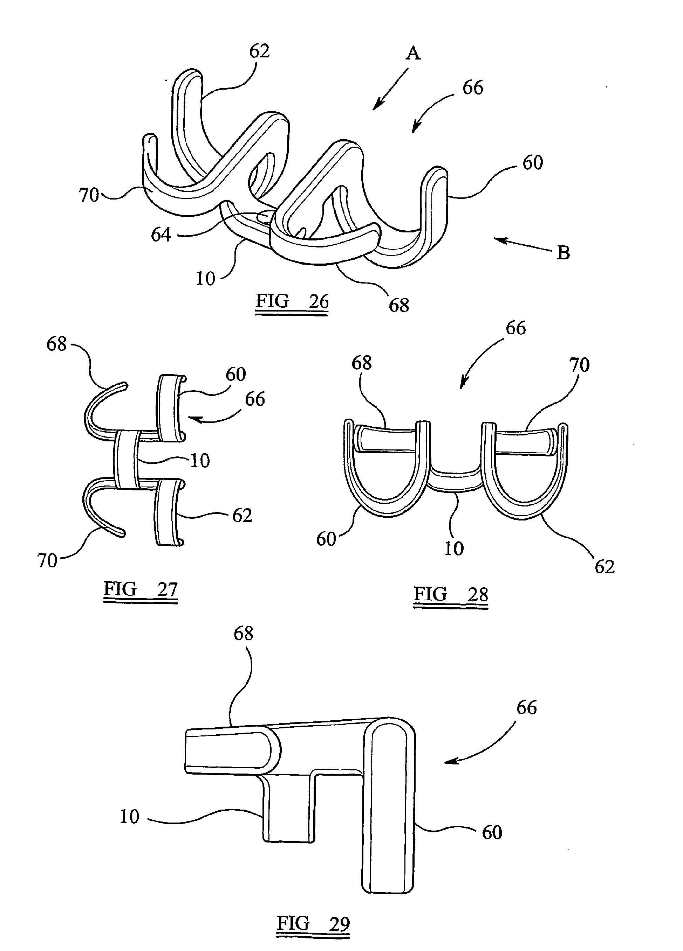

Referring now to Figures 26 - 29, there is shown sixth apparatus 66 for assisting breathing through a person's nose. Similar parts as in previous Figures have been given the same reference numerals for ease of comparison and understanding.

The apparatus 66 is such that it has a third open-ended curved member 68 and a fourth open-ended curved member 70. The first and the third open-ended curved members 60, 68 are for insertion in the first nostril of the person. The second and fourth open-ended curved members 62, 70 are for insertion in the second nostril of the person. The third open-ended curved member 68 extends rearwardly of the first open-ended curved member 60 and is for engaging in a rear part of the first nostril. The fourth open-ended curved member 70 extends rearwardly of the second open-ended curved member 62 and is for engaging in a rear part of the second nostril. The co-operating pairs of open-ended curved members are effective for keeping the first and second nostrils open in a comfortable manner, whilst at the same time ensuring that the apparatus does not become lose during use.

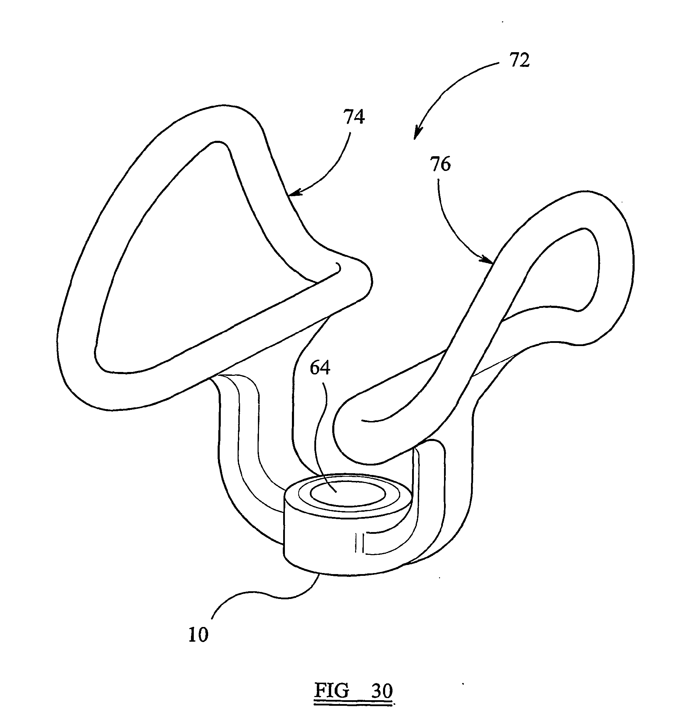

Referring to Figure 30, there is shown seventh apparatus 72 for assisting breathing through a person's nose which is in accordance with the invention. Similar parts as in previous Figures have been given the same reference numerals for ease of comparison and understanding.

The apparatus 72 is such that it has a first member in the form of a first closed non-circular ring member 74. The apparatus 72 also has a second member in the form of a second closed non-circular ring member 76. As shown in Figure 30, both ring members 74, 76 extend at an angle away from the connecting member 10.

The apparatus 72 is able to be moulded quickly and cheaply.

The apparatus 72 may be made in different sizes, for example a small size, a medium size, and a large size.

All of the apparatus of the present invention and shown in the drawings is easily able to be inserted as a comfortable fit in the nose 4. By making the apparatus of silastic plastics material or an equivalent material, the apparatus can be produced to be a soft comfortable fit in the nose 4. In the event that a person wearing the apparatus should be indulging in athletic exercise and should receive a blow on their nose 4, then the apparatus of the invention can be effective to help absorb the pressure of the blow and thus minimise potential damage to the nose 4.

It is to be appreciated that the embodiment of the invention described above with reference to the accompanying drawing has been given by way of example only and that modifications may be effected.

## Claims

1. Apparatus (2) for assisting breathing through a person's nose (4), which apparatus (2) in use comprises a first member (6) for fitting in a first nostril of the nose (4), a second member (8) for fitting in a second nostril of the nose (4), and a connecting member (10) which connects the first and second members (6,8) together and which prevents the first and second members (6,8) being pushed too far into the nose (4), **characterized in that** the first member (6) comprises a strip which defines a closed loop, the second member (8) comprises a strip which defines a closed loop, the connecting member (10) comprises a strip which defines a concave shape, and the strip for the connecting member (10) is larger in size in cross section than the size in cross section of the strips for the first and second members (6,8).

2. Apparatus according to claim 1 in which the first and the second members (6,8) extend upwardly from the connecting member (10) and thereby further into the nostrils of the nose (4) than ends of the connecting member (10) from which the first and second members (6,8) extend.

3. Apparatus according to claim 1 or claim 2 in which the closed loop is not a circular closed loop.

4. Apparatus (2) according to claim 1 in which the first member (6) is a first ring member, and in which the second member (8) is a second ring member.

5. Apparatus (2) according to any one of the preceding claims in which the apparatus (2) is made from a plastics material or a synthetic rubber material.

6. Apparatus (2) according to claim 5 in which the plastics material is a silastic plastics material.

## Patentansprüche

1. Gerät zur Unterstützung der Atmung durch die Nase (4) einer Person, das im Gebrauch ein erstes Element (6), das in einem ersten Nasenloch der Nase (4) anzubringen ist, ein zweites Element (8), das in einem zweiten Nasenloch der Nase (4) anzubringen ist, und ein Verbindungselement (10) umfasst, das das erste und das zweite Element (6, 8) miteinander verbindet und verhindert, dass das erste und das zweite Element (6, 8) zu weit in die Nase (4) geschoben werden, **dadurch gekennzeichnet, dass** das erste Element (6) einen eine geschlossene Schlaufe definierenden Streifen umfasst, das zweite Element (8) einen eine geschlossene Schlaufe definierenden Streifen umfasst, das Verbindungselement (10) einen eine konkave Form definierenden Streifen umfasst und der Streifen für das Verbindungselement (10) im Querschnitt größer ist als die Streifen für das erste und das zweite Element (6, 8) im Querschnitt.

2. Gerät nach Anspruch 1, wobei sich das erste und das zweite Element (6, 8) vom Verbindungselement (10) nach oben erstrecken und dadurch weiter in die Nasenlöcher der Nase (4) hinein als Enden des Verbindungselements (10), von denen sich das erste und das zweite Element (6, 8) erstrecken.

3. Gerät nach Anspruch 1 oder 2, wobei es sich bei der geschlossenen Schlaufe nicht um eine kreisförmige geschlossene Schlaufe handelt.

4. Gerät (2) nach Anspruch 1, wobei das erste Element (6) ein erstes Ringelement ist und wobei das zweite Element (8) ein zweites Ringelement ist.

5. Gerät (2) nach einem der vorhergehenden Ansprüche, wobei das Gerät (2) aus einem Kunststoffmaterial oder Synthesekautschukmaterial hergestellt ist.

6. Gerät (2) nach Anspruch 5, wobei das Kunststoffmaterial ein Silastic-Kunststoffmaterial ist.

## Revendications

1. Appareil (2) pour aider une personne à respirer par le nez (4), lequel appareil (2), pendant l'utilisation, comprend un premier organe (6) destiné à être ajusté dans une première narine du nez (4), un deuxième organe (8) destiné à être ajusté dans une deuxième narine du nez (4), et un organe de connexion (10) qui relie le premier et le deuxième organe (6, 8) l'un à l'autre et qui empêche que le premier et le deuxième organe (6, 8) ne soient poussés trop loin dans le nez (4), **caractérisé en ce que** le premier organe (6) comprend une bande qui définit une boucle fermée, le deuxième organe (8) comprend une bande qui définit une boucle fermée, l'organe de connexion (10) comprend une bande qui définit une forme concave, et la bande pour l'organe de connexion (10) a une dimension en section transversale plus grande que la dimension en section transversale des bandes pour les premier et deuxième organes (6, 8).

2. Appareil selon la revendication 1, dans lequel les premier et deuxième organes (6, 8) s'étendent vers le haut depuis l'organe de connexion (10) et par conséquent davantage dans les narines du nez (4) que des extrémités de l'organe de connexion (10) depuis lesquelles s'étendent les premier et deuxième organes (6, 8).

3. Appareil selon la revendication 1 ou la revendication 2, dans lequel la boucle fermée n'est pas une boucle fermée circulaire.

4. Appareil (2) selon la revendication 1, dans lequel le premier organe (6) est un premier organe annulaire et dans lequel le deuxième organe (8) est un deuxième organe annulaire.

5. Appareil (2) selon l'une quelconque des revendications précédentes, dans lequel l'appareil (2) est fabriqué en un matériau en plastique ou en un matériau en caoutchouc synthétique.

6. Appareil (2) selon la revendication 5, dans lequel le matériau en plastique est un matériau en plastique à base de Silastic.
